# EUROPEAN PATENT SPECIFICATION

(11) **EP 2 470 070 B1**
(45) Date of publication and mention of the grant of the patent: **20.04.2016**
(21) Application number: 10844136.1
(22) Date of filing: 24.09.2010
(51) Int. Cl.: A61B 5/04

(54) **ELECTRICAL METERING OF ACTIVE FIXATION LEAD COUPLING**
ELEKTRISCHE MESSUNG DER KOPPLUNG EINER FIXIERTEN AKTIVEN ELEKTRODE
MESURE ÉLECTRIQUE D'UN COUPLAGE DE FIL CONDUCTEUR À FIXATION ACTIVE

(30) Priority: 30.12.2009 US 650046
(43) Date of publication of application: 04.07.2012
(73) Proprietor: St. Jude Medical Atrial Fibrillation Division Inc., St. Paul, Minnesota 55117-9913 (US)
(72) Inventor: COHEN, Amit, 30500 Binyamina (IL)
(74) Representative: Kramer Barske Schmidtchen Patentanwälte PartG mbB
(86) International application number: PCT/US2010/050241
(87) International publication number: WO 2011/090518

(56) References cited:
- WO-A2-02/24063
- US-A1- 2007 106 289
- US-A1- 2009 276 002
- US-B1- 7 092 766

## Description

### BACKGROUND OF THE INVENTION

### a. Field of the Invention

This invention relates to a system and method for assessing coupling of a lead to tissue. In particular, the invention relates to a system and method in which a capacitance, an inductance, a voltage or a current between an electrode and a fixation element on the lead is used to assess the degree of coupling of the lead to tissue.

### b. Background Art

A number of conventional medial diagnostic and treatment methods exist in which electric signals are applied to tissues in a body. For example, electric signals are commonly used to control heart rates and rhythms in order to maintain or restore a regular heart rate or rhythm in patients suffering from bradyarrhythmias, tachyarrhythmias or fibrillation. The electrical signals are provided to the heart tissue using electrodes disposed on leads that are fed intravenously from an external device or internally from an implanted pacemaker or implantable cardioverter defibrillator (ICD).

Leads are generally attached to the heart tissue using a fixating element such a hook, tines, or a helix or screw extending from the lead. Proper affixation of the lead to the tissue is important to ensure proper placement of the electrodes. Improperly placed electrodes can reduce the useful life of the device by draining power from the device and can also result in less than optimal diagnosis and/or treatment of a condition.

A clinician typically monitors placement and affixation of leads through fluoroscopic imaging. The use of fluoroscopy, however, has several drawbacks. Fluoroscopy requires a subjective judgment on the part of the clinician as to whether the lead has been placed properly and affixed to the tissue. This assessment can be difficult where the leads are being affixed to cardiac tissue because fluoroscopic images are two-dimensional projections and blood and myocardium attenuate x-rays similarly. Fluoroscopic imaging also exposes the patient and clinician to radiation

The inventors herein have recognized a need for a system and method for assessing coupling of a lead to tissue that will minimize and/or eliminate one or more of the above-identified deficiencies.
WO 02/24063A discloses a system and a method for determining tissue contact of an implanted medical device within a body. The system includes a signal generator for supplying a first signal such as a constant current or voltage to the implantable medical device. A resulting voltage or current signal is generated, respectively, and may be sensed as an indication of the impedance of a portion of the body that is proximal to one or more electrodes carried by a lead.

### BRIEF SUMMARY OF THE INVENTION

It is desirable to provide a system and method for assessing the coupling of a lead to tissue.

The invention provides for a system and a corresponding method for assessing coupling of a lead to tissue as per the appended claims.

The above-described system and method are advantageous because they provide a less subjective assessment for assessing the coupling of a lead to tissue. In particular, the use of the capacitance, inductance, voltage or current between the electrode and fixation element provides a measurable quantity indicative of the relative locations of the electrode and fixation element and the degree of coupling of the fixation element to tissue. The inventive system and method also minimize or eliminate the need for fluoroscopic imaging. As a result, exposure of the patient and clinician to radiation is reduced.

The foregoing and other aspects, features, details, utilities and advantages of the present invention will be apparent from reading the following description and claims, and from reviewing the accompanying drawings.

### BRIEF DESCRIPTION OF THE DRAWINGS

Fig. 1 is diagrammatic view of a system for assessing coupling of a lead to tissue in accordance with the present teachings.
Fig. 2 is a diagrammatic view illustrating a portion of the system of Figure 1 at a first time t₁.
Fig. 3 is a diagrammatic view illustrating a portion of the system of Figure 1 at a first time t₂.
Fig. 4 is a flow chart illustrating a method for assessing coupling of a lead to tissue in accordance with one embodiment of the present teachings.
Fig. 5 is a flow chart illustrating a method for assessing coupling of a lead to tissue in accordance with another embodiment of the present teachings.

### DETAILED DESCRIPTION OF THE INVENTION

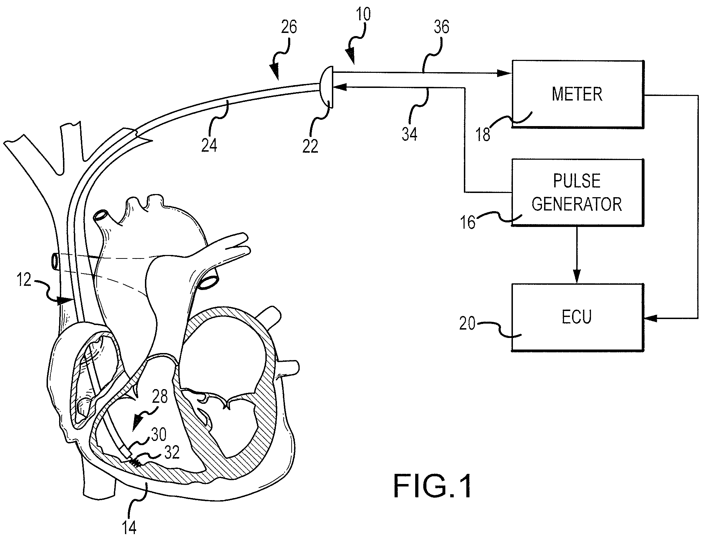

Referring now to the drawings wherein like reference numerals are used to identify identical components in the various views, Fig. 1 illustrates one embodiment of a system 10 for assessing the coupling of a lead 12 to tissue 14 in a body in accordance with one embodiment of the present teachings. In the illustrated embodiment, tissue 14 comprises heart or cardiac tissue. It should be understood, however, that the present invention may be used to assess coupling of lead 12 to a variety of body tissues. In addition to lead 12, system 10 may include a pulse generator 16, a meter 18 and an electronic control unit 20.

Lead 12 is provided for examination, diagnosis and/or treatment of internal body tissues such as tissue 14. In accordance with one embodiment of the present teachings, lead 12 comprises a pacing lead used for controlling heart rhythms. It should be understood, however, that the present invention can be implemented and practiced with a variety of leads. Lead 12 may include a connector or interface 22 and a lead body 24 having a proximal end 26 and a distal 28 end (as used herein, "proximal" refers to a direction toward the end of the catheter near the clinician, and "distal" refers to a direction away from the clinician and (generally) inside the body of a patient). Lead 12 may also include one or more electrodes 30 and a fixation element 32. Lead 12 may also include other conventional components not illustrated herein such as sensors, additional electrodes, and corresponding conductors or leads.

Connector 22 provides mechanical and electrical connections for cables, wires, or other electrical conductors 34, 36 extending from pulse generator 16 and meter 18. Connector may also provide fluid connections and additional mechanical and/or electrical connections depending on the application for lead 12. Connector 22 is conventional in the art and is disposed at a proximal end of lead 12.

Lead body 24 is an elongated, deformable, tubular structure configured for movement with the body (e.g., within the vascular structure). Lead body 24 supports electrodes 30 and fixation element 32 and associated conductors. Lead body 24 may also include additional electronics for signal processing or conditioning. For example, meter 18 may be integrated therein. Lead body 24 may also permit transport, delivery and/or removal of fluids (including irrigation fluids and bodily fluids), medicines and/or surgical tools or instruments. Lead body 24 may be made from biocompatible materials that provide sufficient flexibility and insulation including, for example, silicon and polyurethane. Lead body 24 defines one of more passageways or lumens configured to house and/or transport electrical conductors, fluids or surgical tools. Body 24 extends between proximal end 26 where it is connected to connector 22 and distal end 28 which is disposed proximate the target site in tissue 14. Body 24 may be introduced into a blood vessel or other structure within the body through a conventional introducer. Body 24 may then be steered or guided through the body to a desired location such as tissue 14 with guide wires or other means known in the art.

Electrodes 30 may be provided for a variety of diagnostic and therapeutic purposes including, for example, electrophysiological studies, lead identification and location, pacing, cardiac mapping and ablation. In the illustrated embodiment, electrode 30 comprises a ring electrode disposed at the tip or distal end 28 of lead 12. It should be understood, however, that the number, orientation and purpose of electrodes 30 may vary.

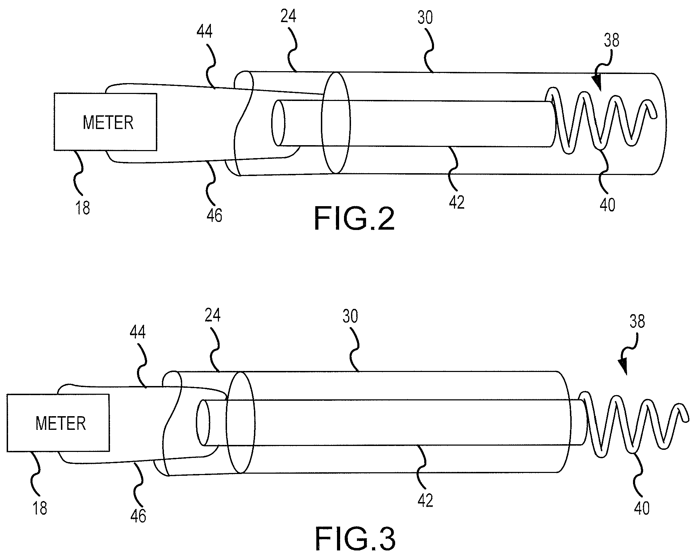

Fixation element 32 is provided to anchor, or secure the position of, the distal end 28 of lead body 24 in tissue 14. Fixation element 32 may be a passive or active fixation element. For example, fixation element may comprise one or more tines extending from lead body 24, a hook, or-as shown in Figures 2-3-a helix 38 or screw. Fixation element 32 may be made from a conducting metal or metal alloy such as Nitinol or stainless steel. Fixation element 32 may provide only an anchoring function or, alternatively, may perform additional functions including use as an electrode. In the helix embodiment illustrated in Figures 2-3, helix 38 includes a distal screw portion 40 terminating at a sharpened point configured to permit entry into tissue 14 and a proximal shaft portion 42 from which screw portion 40 extends. Shaft portion 42 of helix 38 may be rotated within lead body 24 to cause longitudinal movement of helix 38 relative to lead body 24 and electrode 30 and entry into tissue 14.

Pulse generator 16 generates, delivers and controls RF energy used by lead 12. Generator 16 is conventional in the art and may comprise one of the pacemakers sold under the trademark "AFFINITY" by St. Jude Medical, Inc. or a conventional cardiac resynchronization therapy (CRT) device sold under the trademark "FRONTIER" by St. Jude Medical, Inc.. Generator 16 is configured to generate a signal at a predetermined frequency in accordance with one or more user specified parameters (e.g., power, time, etc.) and under the control of various feedback sensing and control circuitry as is know in the art.

Meter 18 is provided to measure the amount of energy between electrode 30 and fixation element 32 and to generate a signal indicative of the amount of energy in response thereto. In one embodiment of the invention, the amount of energy between the electrode 30 and fixation element 32 represents a capacitance and meter 18 comprises a capacitance meter. In another embodiment of the invention, the amount of energy between the electrode 30 and fixation element 32 represents an inductance and meter 18 comprises an inductance meter. In yet other embodiments of the invention, the amount of energy between the electrode 30 and fixation element 32 represents a voltage or a current and meter 18 comprises a volt-meter or ammeter (or amp-meter), respectively. Meter 18 is conventional in the art and may be integrated into lead 12 or an external element, pulse generator 16 or a microprocessor or circuit forming electronic control unit 20. Meter 18 is electrically connected to electrode 30 and fixation element 32 through conductors 44, 46.

ECU 20 is provided to determine a degree of coupling between lead 12 and tissue 14 responsive to the signal received from meter 18. ECU 20 preferably comprises a programmable microprocessor or microcontroller, but may alternatively comprise an application specific integrated circuit (ASIC). ECU 20 may include a central processing unit (CPU) and an input/output (I/O) interface through which ECU 20 may receive a plurality of input signals including signals from meter 18 and generate a plurality of output signals including those used to control generation of electrical signals by electrode 30. In accordance with one aspect of the present invention, ECU 20 may be programmed with a computer program (i.e., software) encoded on a computer storage medium for performing one or more of the above functions.

Referring to Figures 2-3, meter 18 measures the amount of energy between electrode 30 and fixation element 32 as indicated by the voltage or current on conductors 44, 46. The amount of energy between electrode 30 and fixation element 32 changes based on a change in position of fixation element 32. Figure 2 illustrates the relative position of electrode 30 and fixation element 32 at a first time t₁ prior to insertion of fixation element 32 into tissue 14. Figure 3 illustrates the relative position of electrode 30 and fixation element 32 at a second time t₂ after insertion of fixation element 32 into tissue 14. As the fixation element 32 changes position from time t₁ to time t₂, the amount of energy between electrode 30 and fixation element 32 changes thereby providing an indication of the position of fixation element 32 and whether, and to what extent, lead 12 is coupled to tissue 14. This change in the amount of energy is measured by meter 18 and provided to ECU 20. ECU 20 uses the amount of energy measured by meter 18 to assess whether, and to what degree, lead 12 is coupled to tissue 14.

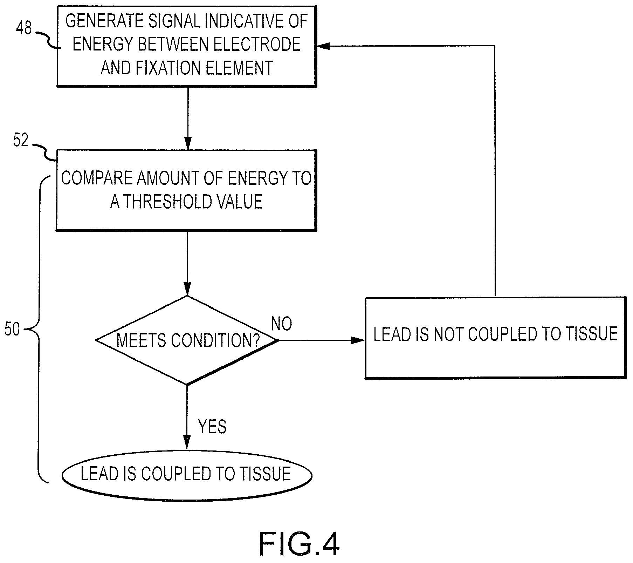

Referring now to Figure 4, a method for assessing coupling of lead 12 to tissue 14 is illustrated. The method may begin with the step 48 of generating a signal indicative of an amount of energy between electrode 30 and fixation element 32. As noted hereinabove, meter 18 measures the amount of energy between electrode 30 and fixation element 32 by measuring voltage or current on conductors 44, 46 and generates a signal indicative of the amount of energy between electrode and fixation element 32. The method continues with the step 50 of determining a degree of coupling between lead 12 and tissue 14 responsive to the signal. Step 50 may include several substeps. In substep 52, ECU 20 may compare the amount of energy indicated by signal to a threshold value. If the amount of energy meets a predetermined condition relative to the threshold value (e.g., if the amount of energy meets, exceeds, or is less than the threshold value depending on the application), ECU 20 may determine that that lead 12 is coupled to tissue 14. If the amount of energy does not meet the predetermined condition relative to the threshold value, ECU 20 may determine that the lead 12 is not coupled to tissue 14.

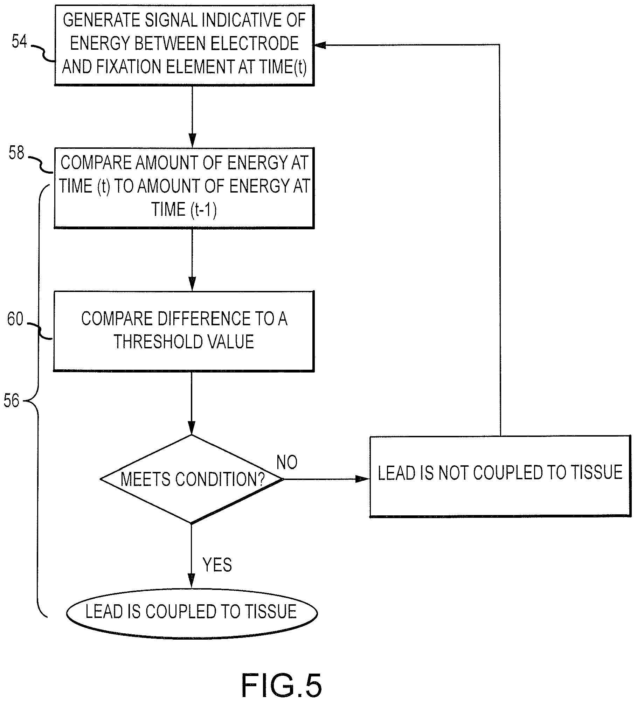

Referring now to Figure 5, a method for assessing coupling of lead 12 to tissue 14 is illustrated. The method may again begin with the step 54 of generating a signal indicative of an amount of energy between electrode 30 and fixation element 32. As noted hereinabove, meter 18 measures the amount of energy between electrode 30 and fixation element 32 by measuring voltage or current on conductors 44, 46 and generates a signal indicative of the amount of energy between electrode and fixation element 32. The method continues with the step 56 of determining a degree of coupling between lead 12 and tissue 14 responsive to the signal. Step 56 may includes several substeps. In substep 58, ECU 20 may compare the amount of energy indicated by signal at a time (*t*) to the amount of energy indicated by the signal at a previous time *(t-1)* by, for example calculating a difference between the two values. In substep 60, ECU 20 may compare the difference to a threshold value. If the difference in the amounts of energy meets a predetermined condition relative to the threshold value (e.g., if the difference meets, exceeds, or is less than the threshold value depending on the application), ECU 20 may determine that that lead 12 is coupled to tissue 14. If the difference in the amounts of energy does not meet the predetermined condition relative to the threshold value, ECU 20 may determine that the lead 12 is not coupled to tissue 14.

A system and method in accordance with the present teachings offers one or more of a number of advantages. The system and method provide a less subjective assessment for assessing the coupling of a lead 12 to tissue 14. In particular, the use of the amount of energy between the electrode 30 and fixation element 32 provides a measurable quantity indicative of the relative locations of the electrode 30 and fixation element 32 and the degree of coupling of the fixation element 32 to tissue. The inventive system and method also minimize or eliminate the need for fluoroscopic imaging. As a result, exposure of the patient and clinician to radiation is reduced.

Although several embodiments of this invention have been described above with a certain degree of particularity, those skilled in the art could make numerous alterations to the disclosed embodiments without departing from the scope of this invention. All directional references (e.g., upper, lower, upward, downward, left, right, leftward, rightward, top, bottom, above, below, vertical, horizontal, clockwise and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not as limiting. Changes in detail or structure may be made without departing from the invention as defined in the appended claims.

## Claims

1. A system (10) for assessing coupling of a lead (12) to tissue (14), comprising:
a lead body (24) including:
an electrode (30) disposed proximate a distal end (28) of said lead body (24);
a fixation element (32) disposed near said distal end (28) of said lead body (24), said fixation element (32) configured to anchor said distal end (28) of said lead body (24) in said tissue (14);
a meter (18) configured to generate a signal indicative of a capacitance, inductance, voltage or current between said electrode (30) and said fixation element (32), wherein the meter (18) includes, respectively, a capacitance meter, an inductance meter, a volt-meter or an amp-meter; and
an electronic control unit (20) configured to determine a degree of coupling between said lead (12) and said tissue (14) responsive to said signal,
wherein said electronic control unit (20) is further configured, in determining said degree of coupling, to calculate a difference between the values of said capacitance, inductance, voltage or current at first and second times and to determine whether the lead is coupled to tissue or not by determining whether said difference meets a predetermined condition relative to a threshold value.

2. The system (10) of claim 1 wherein said electrode comprises a ring electrode.

3. The system (10) of claim 1 wherein said fixation element (32) comprises a helix (38).

4. The system (10) of any one of the preceding claims wherein said electronic control unit (20) is further configured, in determining said degree of coupling, to compare said difference in said capacitance, inductance, voltage or current to a threshold value.

5. A method for assessing coupling of a lead (12) to tissue (14), comprising the steps of:
generating a signal indicative of a capacitance, inductance, voltage or current between an electrode (30) disposed proximate a distal end (28) of a lead body (24) and a fixation element (32) disposed near said distal end (28) of said lead body (24), said fixation element (32) configured to anchor said distal end (28) of said lead body (24) in said tissue (14), wherein the signal is generated by a meter (18), wherein the meter (18) includes, respectively, a capacitance meter, an inductance meter, a volt-meter or an amp-meter; and
determining a degree of coupling between said lead (12) and said tissue (14) responsive to said signal, wherein said determining step includes the substep of calculating a difference between the values of said capacitance, inductance, voltage or current at first and second times and the substep of determining whether the lead is coupled to tissue or not by determining whether said difference meets a predetermined condition relative to a threshold value.

6. The method of claim 5 wherein said electrode (30) comprises a ring electrode.

7. The method of claim 5 wherein said fixation element (32) comprises a helix (38).

8. The method of claim 5 wherein said determining step further includes the substep of comparing said difference in said capacitance, inductance, voltage or current to a threshold value.

## Patentansprüche

1. System (10) zur Beurteilung einer Kopplung einer Führung (12) mit einem Gewebe (14), mit:
einem Führungskörper (24), der aufweist:
eine Elektrode (30), die benachbart zu einem distalen Ende (28) des Führungskörpers (24) ist;
ein Fixierungselement (32), das sich in der Nähe des distalen Endes (28) des Führungskörpers (24) befindet, wobei das Fixierungselement (32) ausgebildet ist zur Verankerung des distalen Endes (28) des Führungskörpers (24) in dem Gewebe (14);
einem Messgerät (18), das ausgebildet ist zur Erzeugung eines Signals, das kennzeichnend ist für eine Kapazität, Induktivität, Spannung oder einen Strom zwischen der Elektrode (30) und dem Fixierungselement (32), wobei das Messgerät (18) jeweils ein Kapazitätsmessgerät, ein Induktivitätsmessgerät, ein Spannungsmessgerät oder ein Strommessgerät aufweist; und
einer elektronischen Steuerungseinheit (20), die ausgebildet ist zum Bestimmen eines Kopplungsgrads zwischen der Führung (12) und dem Gewebe (14) in Antwort auf das Signal,
wobei die elektronische Steuerungseinheit (20) ferner ausgebildet ist bei der Beurteilung des Kopplungsgrads eine Differenz zu berechnen zwischen den Werten der Kapazität, Induktivität, Spannung oder des Strom bei einem ersten und zweiten Zeitpunkt, und zum Bestimmen, ob die Führung mit dem Gewebe gekoppelt ist, oder nicht, indem bestimmt wird, ob die Differenz eine vorbestimmte Bedingung relativ zu einem Schwellenwert erfüllt.

2. System (10) nach Anspruch 1, bei dem die Elektrode eine Ringelektrode aufweist.

3. System (10) nach Anspruch 1, bei dem das Fixierungselement (32) eine Spirale (38) aufweist.

4. System (10) nach einem der vorangegangenen Ansprüche, bei dem die elektronische Steuerungseinheit (20) ferner ausgebildet ist bei der Beurteilung des Kopplungsgrads die Differenz der Kapazität, Induktivität, Spannung und des Stroms mit einem Schwellenwert zu vergleichen.

5. Verfahren zum Beurteilen einer Kopplung einer Führung (12) mit einem Gewebe (14), mit den Schritten:
Erzeugen eines Signals, das kennzeichnend ist für eine Kapazität, Induktivität, Spannung oder einen Strom zwischen einer Elektrode (30), die sich benachbart zu einem distalen Ende (28) eines Führungskörpers (24) befindet, und einem Fixierungselement (32), das sich in der Nähe des distalen Endes (28) des Führungskörpers (24) befindet, wobei das Fixierungselement (32) ausgebildet ist zur Verankerung des distalen Endes (28) des Führungskörpers (24) in dem Gewebe (14), wobei das Signal durch ein Messgerät (18) erzeugt wird, wobei das Messgerät (18) jeweils ein Kapazitätsmessgerät, ein Induktivitätsmessgerät, ein Spannungsmessgerät oder ein Strommessgerät aufweist; und
Bestimmen eines Kopplungsgrades zwischen der Führung (12) und dem Gewebe (14) in Antwort auf das Signal, wobei der Bestimmungsschritt den Nebenschritt des Berechnens einer Differenz zwischen den Werten der Kapazität, Induktivität, Spannung und des Stroms zu einem ersten und zweiten Zeitpunkt aufweist, und den Nebenschritt des Bestimmens, ob die Führung mit dem Gewebe gekoppelt ist, oder nicht, indem bestimmt wird, ob die Differenz eine vorbestimmte Bedingung relativ zu einem Schwellenwert erfüllt.

6. Verfahren nach Anspruch 5, bei dem die Elektrode (30) eine Ringelektrode aufweist.

7. Verfahren nach Anspruch 5, bei dem das Fixierungselement (32) eine Spirale (38) aufweist.

8. Verfahren nach Anspruch 5, bei dem der Bestimmungsschritt ferner den Nebenschritt aufweist des Vergleichens der Differenz der Kapazität, Induktivität, Spannung oder des Stroms mit einem Schwellenwert.

## Revendications

1. Système (10) pour évaluer le couplage d'un fil conducteur (12) avec un tissu (14), comprenant:
un corps de fil conducteur (24), comprenant:
une électrode (30) disposée à proximité d'une extrémité distale (28) dudit corps de fil conducteur (24);
un élément de fixation (32) disposé à proximité de ladite extrémité distale (28) dudit corps de fil conducteur (24), ledit élément de fixation (32) étant configuré pour ancrer ladite extrémité distale (28) dudit corps de fil conducteur (24) dans ledit tissu (14);
un dispositif de mesure (18) configuré pour générer un signal indicatif d'une capacité, d'une inductance, d'une tension ou d'un courant entre ladite électrode (30) et ledit élément de fixation (32), dans lequel le dispositif de mesure (18) comprend, respectivement, un dispositif de mesure de capacité, un dispositif de mesure d'inductance, un voltmètre ou un ampèremètre; et
une unité de commande électronique (20) configurée pour déterminer un degré de couplage entre ledit fil conducteur (12) et ledit tissu (14) qui réagit audit signal,
dans lequel ladite unité de commande électronique (20) est en outre configurée, lors de la détermination dudit degré de couplage, pour calculer une différence entre les valeurs de ladite capacité, de ladite inductance, de ladite tension ou dudit courant à des premier et deuxième instants, et pour déterminer si le fil conducteur est couplé ou non à un tissu en déterminant si ladite différence satisfait à une condition prédéterminée par rapport à une valeur de seuil.

2. Système (10) selon la revendication 1, dans lequel ladite électrode comprend une électrode annulaire.

3. Système (10) selon la revendication 1, dans lequel ledit élément de fixation (32) comprend une hélice (38).

4. Système (10) selon l'une quelconque des revendications précédentes, dans lequel ladite unité de commande électronique (20) est en outre configurée, lors de la détermination dudit degré de couplage, pour comparer ladite différence dans ladite capacité, ladite inductance, ladite tension ou ledit courant par rapport à une valeur de seuil.

5. Procédé pour évaluer le couplage d'un fil conducteur (12) avec un tissu (14), comprenant les étapes suivantes:
générer un signal indicatif d'une capacité, d'une inductance, d'une tension ou d'un courant entre une électrode (30) disposée à proximité d'une extrémité distale (28) d'un corps de fil conducteur (24) et un élément de fixation (32) disposé à proximité de ladite extrémité distale (28) dudit corps de fil conducteur (24), ledit élément de fixation (32) étant configuré pour ancrer ladite extrémité distale (28) dudit corps de fil conducteur (24) dans ledit tissu (14), dans lequel le signal est généré par un dispositif de mesure (18), dans lequel le dispositif de mesure (18) comprend, respectivement, un dispositif de mesure de capacité, un dispositif de mesure d'inductance, un voltmètre ou un ampèremètre; et
déterminer un degré de couplage entre ledit fil conducteur (12) et ledit tissu (14) qui réagit au signal,
dans lequel ladite étape de détermination comprend l'étape secondaire consistant à calculer une différence entre les valeurs de ladite capacité, de ladite inductance, de ladite tension ou dudit courant à des premier et deuxième instants ainsi que l'étape secondaire consistant à déterminer si le fil conducteur est couplé ou non à un tissu en déterminant si ladite différence satisfait à une condition prédéterminée par rapport à une valeur de seuil.

6. Procédé selon la revendication 5, dans lequel ladite électrode (30) comprend une électrode annulaire.

7. Procédé selon la revendication 5, dans lequel ledit élément de fixation (32) comprend une hélice (38).

8. Procédé selon la revendication 5, dans lequel ladite étape de détermination comprend en outre l'étape secondaire consistant à comparer ladite différence dans ladite capacité, ladite inductance, ladite tension ou ledit courant par rapport à une valeur de seuil.
